# FASCICULE DE BREVET EUROPEEN

(11) **EP 4 073 498 B1**
(45) Date de publication et mention de la délivrance du brevet: **13.09.2023**
(21) Numéro de dépôt: 21701818.3
(22) Date de dépôt: 01.02.2021
(51) Int. Cl.: G01N 27/327, A61B 5/145, G01N 33/00

(54) **PROCÉDÉ ET SYSTÈME D'ÉTALONNAGE D'UN CAPTEUR CHIMIQUE NON SÉLECTIF**
VERFAHREN UND SYSTEM FÜR DIE KALIBRIERUNG EINE CHEMISCHEN NICHT-SELEKTIVEN SENSORS
METHOD AND SYSTEM FOR CALIBRATING A CHEMICAL NON-SELECTIVE SENSOR

(30) Priorité: 07.02.2020 FR 2001251
(43) Date de publication de la demande: 19.10.2022
(73) Titulaire: ELLONA, 31400 Toulouse (FR)
(72) Inventeur: ROMANYTSIA, Ivan, 31300 TOULOUSE (FR)
(74) Mandataire: Argyma
(86) Numéro de dépôt international: PCT/EP2021/052288
(87) Numéro de publication internationale: WO 2021/156193

(56) Documents cités:
- EP-A2- 2 327 984
- EP-B1- 2 327 984
- US-A1- 2009 156 924
- US-A1- 2009 192 751

## Description

### DOMAINE TECHNIQUE

La présente invention concerne le domaine de l'étalonnage d'un capteur chimique non sélectif.

La sélectivité d'un capteur chimique est définie par son aptitude à déterminer un signal chimique de la quantité d'un unique composé chimique d'un milieu chimique à l'exception des autres. On distingue par ailleurs un composé chimique d'un autre composé chimique d'une part par son type, c'est-à-dire sa composition chimique, et d'autre part par sa nature, c'est-à-dire ses propriétés physico-chimiques.

Le brevet européen EP2 327 984A décrit un procédé d'étalonnage d'un capteur de glucose, dans lequel une fonction de conversion est calculée à partir de paires de valeurs mesurées, qui est régulièrement mise à jour avec de nouvelles données adaptées.

De manière connue, un capteur chimique non sélectif, tel qu'un capteur semi-conducteur, est configuré pour déterminer un signal de mesure de la quantité d'un groupe de composés chimiques d'un milieu chimique, telle que la concentration en composés organiques volatils, d'abréviation « COV », dans l'air. Par COV, on désigne à titre d'exemples les polluants émis par les moyens de transport, les imprimantes, les radiateurs, le tabagisme, la combustion de bougies ou encore les émissions émises par le mûrissement des fruits et légumes. Pour cela, un capteur semi-conducteur, également nommé « capteur MOX » ou « capteur MOS », comprend une couche d'oxyde métallique chauffée par un élément chauffant sur laquelle viennent se fixer les COV, ce phénomène étant connu de l'homme du métier sous le terme « d'adsorption ». Un tel capteur semi-conducteur comprend en outre des électrodes de mesure de la conductivité électrique de la couche d'oxyde métallique, qui est fonction de la quantité de COV adsorbés, ce qui permet de déterminer la concentration en COV dans l'air à partir d'une fonction de conversion propre au capteur semi-conducteur. La détermination de la concentration en COV de l'air peut notamment être utilisée pour mesurer la qualité de l'air intérieur d'un bureau, d'un logement, d'une école ou d'une zone de conditionnement et/ou mûrissement de fruits et légumes, ou encore pour mesurer la qualité de l'air extérieur à proximité d'un axe de transport, tel qu'un axe routier ou un aéroport.

De manière connue, un capteur chimique non sélectif doit être étalonné avant sa première utilisation. Pour cela, en reprenant l'exemple précédent du capteur semi-conducteur, le capteur semi-conducteur est placé dans plusieurs milieux étalons dont la concentration en COV est connue. L'écart entre la concentration en COV mesurée par le capteur semi-conducteur et la concentration en COV théorique de chaque milieu étalon est corrigé en ajustant la fonction de conversion entre la conductivité électrique et la concentration en COV propre au capteur semi-conducteur.

Dans les faits, la réponse d'un capteur semi-conducteur dérive au cours de son utilisation, notamment à cause de l'obstruction progressive des pores de la couche d'oxyde métallique due à l'adsorption des COV qui modifie sa conductivité électrique. Un tel capteur semi-conducteur doit ainsi être régulièrement étalonné pour que la concentration en COV mesurée reste fiable et précise. Ceci nécessite de désinstaller le capteur semi-conducteur pour le placer dans les milieux étalons puis de réinstaller ledit capteur semi-conducteur dans le bureau, ce qui est coûteux en temps. En outre, en cas d'oubli ou de retard pour réaliser l'étalonnage ou encore de dérèglement imprévu du capteur semi-conducteur, une concentration en COV faussée peut être mesurée sans le savoir.

L'invention vise ainsi un procédé et un système d'étalonnage d'un capteur chimique non sélectif permettant d'éliminer au moins certains de ces inconvénients.

### PRESENTATION DE L'INVENTION

L'invention concerne un procédé d'étalonnage d'au moins un capteur chimique non sélectif, dit « capteur à étalonner », au moyen d'au moins un capteur de référence, ledit capteur à étalonner et ledit capteur de référence étant au contact d'un même milieu chimique, ledit capteur à étalonner étant configuré pour déterminer un signal chimique de la quantité d'un groupe de composés chimiques dans ledit milieu chimique, ledit capteur de référence étant configuré pour déterminer un signal de référence dont la variation temporelle est corrélée à celle dudit signal chimique, ledit capteur à étalonner comprenant :
- un élément de mesure d'un signal électrique, qui est fonction de la quantité dudit groupe de composés chimiques dans ledit milieu chimique, et
- un élément de calcul dudit signal chimique à partir d'une fonction de conversion f propre audit capteur à étalonner et configurée pour associer audit signal électrique U mesuré le signal chimique S1 correspondant suivant la relation suivante : S1 = f(U).

L'invention est remarquable en ce que le procédé comprend :
- une étape de mesure, pendant une période de référence, d'un premier signal chimique S1_{P1} au moyen du capteur à étalonner et d'un premier signal de référence S2_{P1} au moyen du capteur de référence,
- une étape de détermination d'une première fonction de régression g_{P1} définie de la manière suivante : S2_{P1} = g_{P1}(S1_{P1}),
- au moins une étape de mesure, pendant une période de test postérieure à la période de référence, d'un deuxième signal chimique S1_{P2} au moyen du capteur à étalonner et d'un deuxième signal de référence S2_{P2} au moyen du capteur de référence,
- au moins une étape de détermination d'une deuxième fonction de régression g_{P2} définie de la manière suivante : S2_{P2} = g_{P2}(S1_{P2}),
- au moins une étape de calcul d'un écart entre la première fonction de régression et la deuxième fonction de régression et
- lorsque l'écart est supérieur à un écart de référence, au moins une étape de détermination d'une fonction de conversion optimisée afin que la deuxième fonction de régression soit sensiblement égale à la première fonction de régression, de manière à étalonner le capteur à étalonner.

Le procédé selon l'invention est défini dans la revendication 1. Grâce à l'invention, un capteur chimique non sélectif peut être étalonné directement lors de l'acquisition de mesures d'intérêt, c'est-à-dire dans le milieu chimique dont on souhaite déterminer la quantité d'un groupe de composés chimiques. Il n'est donc pas nécessaire de placer le capteur à étalonner dans un ou plusieurs milieux étalons de composition chimique connue. L'étalonnage est ainsi plus simple, plus rapide et plus pratique à mettre en oeuvre, mais également plus pertinent car réalisé directement dans le milieu chimique d'intérêt.

Un tel procédé d'étalonnage est en outre fiable car basé sur des comparaisons de mesures du capteur à étalonner et d'un capteur de référence jouant le rôle de capteur étalon, dont les mesures sont considérées comme valides et corrélées temporellement aux mesures du capteur à étalonner. Chaque comparaison est en outre réalisée sur la base de mesures effectuées sur la même période de temps et dans le même milieu chimique, ce qui renforce la fiabilité. Par ailleurs, un tel procédé d'étalonnage est précis car la première comparaison est réalisée lors d'une période de référence où une mesure du capteur chimique non sélectif est réputée valide, à la manière d'une phase de calibration. La comparaison lors d'une période de test est en outre répétable quand et aussi souvent qu'on le souhaite, par exemple de manière périodique.

Selon un aspect, le procédé d'étalonnage est mis en oeuvre pour un unique capteur à étalonner et un unique capteur de référence. Un tel procédé est adapté pour l'étalonnage d'un seul capteur.

Selon un autre aspect, le procédé d'étalonnage est mis en oeuvre pour une pluralité de capteurs à étalonner et au moins un capteur de référence, chaque capteur à étalonner étant étalonné au moyen d'un unique capteur de référence, de préférence identique pour tous les capteurs à étalonner. Un tel procédé est adapté pour l'étalonnage d'une barrette de capteurs. L'utilisation d'un unique capteur de référence permet avantageusement de limiter l'encombrement.

Selon un aspect de l'invention, le procédé d'étalonnage est mis en oeuvre pour une pluralité de périodes de test postérieures à la période de référence, ledit procédé comprenant pour chaque période de test :
- une étape de mesure, pendant la période de test, d'un deuxième signal chimique S1_{P2} au moyen du capteur à étalonner et d'un deuxième signal de référence S2_{P2} au moyen du capteur de référence,
- une étape de détermination d'une deuxième fonction de régression g_{P2} définie de la manière suivante : S2_{P2} = g_{P2}(S1_{P2}),
- une étape de calcul d'un écart entre la première fonction de régression et la deuxième fonction de régression et
- lorsque l'écart est supérieur à un écart de référence, une étape de détermination d'une fonction de conversion optimisée afin que la deuxième fonction de régression soit sensiblement égale à la première fonction de régression, de manière à étalonner le capteur à étalonner.

De manière avantageuse, la première fonction de régression calculée pour la période de référence n'a pas à être recalculée, ce qui permet un gain de temps. La première fonction de régression sert ainsi de témoin pour toutes les périodes de test suivantes, ce qui rend le procédé fiable et précis, car la fonction de conversion optimisée est toujours obtenue à partir de la même première fonction de régression. En outre, un tel procédé d'étalonnage peut ainsi aisément être reproduit de nombreuses fois, de préférence de manière périodique pour prévenir la dérive du capteur à étalonner.

Selon un aspect de l'invention, le capteur à étalonner se présente sous la forme d'un capteur de COV, afin de réaliser des mesures de polluants dans l'air.

De préférence, le capteur à étalonner se présente sous la forme d'un capteur semi-conducteur ou d'un capteur électrochimique ou d'un capteur à photoionisation. Le choix de la nature du capteur est avantageusement réalisé en fonction des composés chimique dont on souhaite mesurer la quantité. Pour mesurer une quantité de divers composés chimiques, un capteur semi-conducteur représente une solution économique et précise. Pour mesurer une quantité de composés chimiques plus ciblés, un capteur électrochimique représente une solution fiable et précise. Pour mesurer une quantité de composés chimiques ionisables, un capteur à photoionisation est plus adapté et précis.

Selon un aspect de l'invention, le capteur de référence se présente sous la forme d'un capteur de dioxyde de carbone, afin de mesurer la quantité de dioxyde de carbone dans un espace clos occupé, cette quantité étant avantageusement corrélée à l'occupation de l'espace, et donc à la quantité de COV émis dans ledit espace. De préférence, le capteur de référence se présente sous la forme d'un capteur infrarouge non dispersif, connu sous l'abréviation « NDIR ».

Selon un autre aspect de l'invention, le capteur de référence se présente sous la forme d'un capteur d'intensité sonore, afin de mesurer par exemple le bruit émis à proximité d'un axe routier, ce bruit étant avantageusement corrélé à la circulation sur l'axe routier, et donc à la quantité de COV émis par ladite circulation sur l'axe routier.

De préférence, le capteur de référence se présente sous la forme d'un capteur autocalibré, dont les mesures sont avantageusement valides à tout instant et ne dérivent pas au cours de son utilisation. Un capteur de référence autocalibré forme ainsi un capteur étalon fiable et précis à la fois pour la période de référence et pour la ou les période(s) de test.

Selon un aspect, la période de référence est comprise entre un jour et un mois, de préférence de l'ordre d'une semaine. De préférence, la période de test est comprise entre un jour et un mois, de préférence de l'ordre d'une semaine, de préférence encore égale à la période de référence. De manière avantageuse, une telle période de référence et une telle période de test sont suffisamment longues pour caractériser toute la plage de variation de la quantité de composés chimiques dans le milieu chimique, et suffisamment courtes pour limiter le stockage des données.

Selon un aspect préféré, le premier signal chimique comprend une pluralité de points de mesure espacés d'un pas de mesure compris entre 1min et 4h, de préférence de l'ordre de 1h. De préférence, le premier signal de référence comprend un même nombre de points de mesure que le premier signal chimique et espacés du même pas de mesure. De préférence également, le deuxième signal chimique comprend une pluralité de points de mesure espacés d'un pas de mesure compris entre 1min et 4h, de préférence de l'ordre de 1h. Préférentiellement, le deuxième signal de référence comprend un même nombre de points de mesure que le deuxième signal chimique et espacés du même pas de mesure. De manière avantageuse, les fonctions de régression obtenues comportent suffisamment de points pour être représentatives et précises, tout en limitant le stockage de données.

De préférence, la première fonction de régression est polynomiale, de préférence affine, de préférence encore linéaire. De préférence également, la deuxième fonction de régression est polynomiale, de préférence affine, de préférence encore linéaire. De telles fonctions de régression sont avantageusement simples à déterminer et à exploiter.

Selon un aspect de l'invention, l'écart vérifie la relation suivante : ε = Max (|S_{P1}-S1_{P2}|) / S1_{P1} tel que S2_{P1}= S2_{P2}. Un tel écart est avantageusement simple à déterminer et représentatif de l'éventuelle dérive du capteur à étalonner.

De préférence, l'écart de référence est compris entre 0,01 et 0,1, de préférence de l'ordre de 0,05, ce qui permet d'éviter toute dérive importante tout en évitant de devoir réaliser une micro-correction peu pertinente.

Selon un aspect de l'invention, le deuxième signal chimique S2_{P2} étant déterminé à partir d'un deuxième signal électrique U_{P2}, la fonction de conversion optimisée f* vérifie la relation suivante : g_{P2}(f*(U_{P2})) = g_{P1}(S1_{P1}). Une telle fonction de conversion optimisée permet d'étalonner le capteur de manière à ce que pour une quantité donnée de composés chimiques, le capteur à étalonner fournisse le même signal chimique que lors de la période de référence.

L'invention concerne également un système d'étalonnage pour la mise en oeuvre du procédé d'étalonnage tel que décrit précédemment, ledit système comprenant au moins un capteur chimique non sélectif, dit « capteur à étalonner », et au moins un capteur de référence configurés pour être au contact d'un même milieu chimique et un module de calcul relié au capteur à étalonner et au capteur de référence, ledit capteur à étalonner étant configuré pour déterminer un signal chimique de la quantité d'un groupe de composés chimiques dans ledit milieu chimique, en particulier un premier signal chimique S1_{P1} pendant une période de référence et un deuxième signal chimique S1_{P2} pendant une période de test, ledit capteur de référence étant configuré pour déterminer un signal de référence dont la variation temporelle est corrélée à celle dudit signal chimique, en particulier un premier signal de référence S2_{P1} pendant ladite période de référence et un deuxième signal de référence S2_{P2} pendant ladite période de test, ledit capteur à étalonner comprenant :
- un élément de mesure d'un signal électrique, qui est fonction de la quantité dudit groupe de composés chimiques dans ledit milieu chimique, et
- un élément de calcul dudit signal chimique à partir d'une fonction de conversion f propre audit capteur à étalonner et configurée pour associer audit signal électrique U mesuré le signal chimique S1 correspondant suivant la relation suivante : S1 = f(U),
- ledit module de calcul étant configuré pour :
   - déterminer une première fonction de régression g_{P1} et une deuxième fonction de régression g_{P2} définies de la manière suivante : S2_{P1} = g_{P1}(S1_{P1}) et S2_{P2}=g_{P2}(S1_{P2})
   - calculer un écart entre la première fonction de régression et la deuxième fonction de régression et
   - lorsque l'écart est supérieur à un écart de référence, déterminer une fonction de conversion optimisée afin que la deuxième fonction de régression soit sensiblement égale à la première fonction de régression, de manière à étalonner le capteur à étalonner.

De manière avantageuse, un tel système d'étalonnage est peu encombrant et peut être installé de manière simple et pratique directement dans le milieu physique que mesure le capteur à étalonner. Un tel système d'étalonnage ne nécessite ainsi pas de déplacer le capteur à étalonner ni d'intervention humaine, ce qui procure un gain en temps et en ressources humaines.

Selon un aspect, le système d'étalonnage comprend un unique capteur à étalonner et un unique capteur de référence. Selon un autre aspect, le système d'étalonnage comprend une pluralité de capteurs à étalonner et au moins un capteur de référence, chaque capteur à étalonner étant étalonné au moyen d'un unique capteur de référence, de préférence identique pour tous les capteurs à étalonner. Un tel système d'étalonnage permet avantageusement l'étalonnage d'une barrette de capteurs, de préférence avec un unique capteur de référence pour limiter l'encombrement.

L'invention concerne en outre un programme d'ordinateur mettant en oeuvre le procédé d'étalonnage tel que décrit précédemment.

### PRESENTATION DES FIGURES

L'invention sera mieux comprise à la lecture de la description qui va suivre, donnée uniquement à titre d'exemple, et se référant aux dessins annexés donnés à titre d'exemples non limitatifs, dans lesquels des références identiques sont données à des objets semblables et sur lesquels :
[Fig. 1] est une représentation schématique fonctionnelle d'un capteur à étalonner ;
[Fig. 2] est une représentation schématique fonctionnelle d'un système d'étalonnage du capteur à étalonner de la figure 1 selon une forme de réalisation de l'invention et lors d'une période de référence ;
[Fig. 3] est une représentation schématique fonctionnelle du système d'étalonnage de la figure 2, lors d'une période de test ;
[Fig. 4] est une représentation schématique des étapes d'un procédé d'étalonnage selon un mode de réalisation de l'invention ;
[Fig. 5] et
[Fig. 6] sont des représentations schématiques fonctionnelles d'un module de calcul du système d'étalonnage de la figure 2, respectivement lors de la période de référence et lors de la période de test ;
[Fig. 7] est une représentation des signaux physiques du capteur à étalonner et d'un capteur de référence du système d'étalonnage de la figure 2, lors de la période de référence et lors de la période de test ;
[Fig. 8] est une représentation schématique des fonctions de régression, pour la période de référence et pour la période de test, entre le signal chimique du capteur à étalonner et celui du capteur de référence du système d'étalonnage de la figure 2 ;
[Fig. 9] est une représentation schématique fonctionnelle du système d'étalonnage selon une autre forme de réalisation de l'invention et
[Fig. 10] est une représentation des signaux physiques du capteur à étalonner et du capteur de référence du système d'étalonnage de la figure 9, lors de la période de référence et lors de la période de test.

Il faut noter que les figures exposent l'invention de manière détaillée pour mettre en oeuvre l'invention, lesdites figures pouvant bien entendu servir à mieux définir l'invention le cas échéant.

### DESCRIPTION DETAILLEE DE L'INVENTION

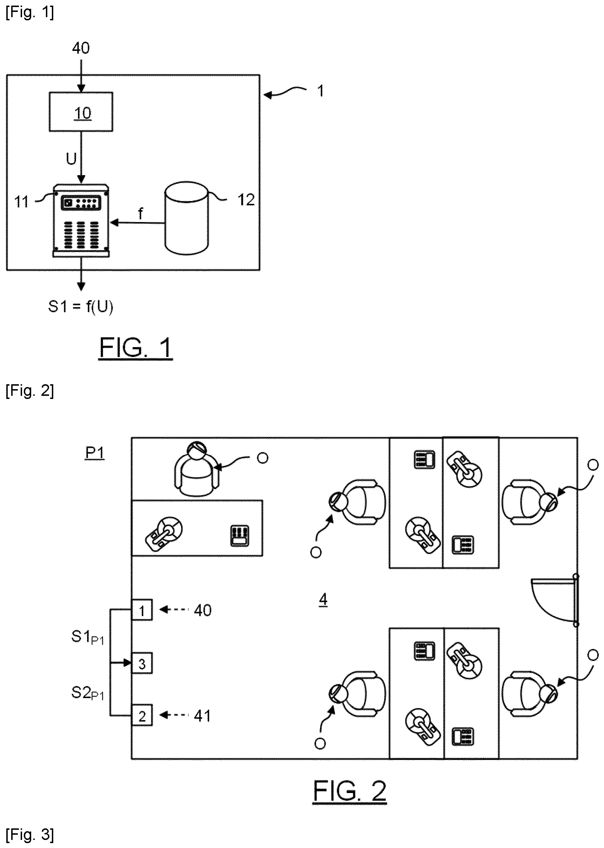

De manière connue et en référence à la figure 1, un capteur chimique non sélectif 1 est configuré pour déterminer un signal chimique S1 de la quantité d'un groupe de composés chimiques 40 dans un milieu chimique. On définit ici la sélectivité d'un capteur chimique par son aptitude à déterminer un signal chimique de la quantité d'un unique composé chimique d'un milieu chimique à l'exception des autres. On distingue par ailleurs un composé chimique d'un autre composé chimique d'une part par son type, c'est-à-dire sa composition chimique, et d'autre part par sa nature, c'est-à-dire ses propriétés physico-chimiques.

Toujours en référence à la figure 1, un capteur chimique non sélectif 1 comprend :
- un élément de mesure 10 d'un signal électrique U, qui est fonction de la quantité du groupe de composés chimiques 40 dans le milieu chimique, et
- un élément de calcul 11 du signal chimique S1 à partir d'une fonction de conversion f configurée pour associer au signal électrique U mesuré le signal chimique S1 correspondant suivant la relation suivante : S1 = f(U). La fonction de conversion f est propre au capteur 1 et provient d'une base de données 12 intégrée ou non à l'élément de calcul 11.

A titre d'exemple de capteur chimique non sélectif, on considère par la suite un capteur semi-conducteur 1, également nommé « capteur MOX » ou « capteur MOS », configuré pour déterminer la concentration S1 de composés organiques volatils 40, d'abréviation « COV », dans l'air intérieur d'un bureau 4, comme illustré sur les figures 2 et 3. Un tel capteur semi-conducteur 1 comprend un élément de mesure 10 comprenant une couche d'oxyde métallique, un élément chauffant et des électrodes de mesure. La couche d'oxyde métallique est chauffée par l'élément chauffant de manière à ce que viennent se fixer les COV 40, ce phénomène étant connu de l'homme du métier sous le terme « d'adsorption ». L'élément de mesure 10, et plus précisément les électrodes de mesure, est configuré pour mesurer une conductivité électrique U de la couche d'oxyde métallique, qui est fonction de la quantité de COV adsorbés et forme le signal électrique. Un signal chimique se présentant sous la forme d'une concentration S1 en COV 40 est ensuite déterminé à partir de la conductivité électrique U mesurée via la fonction de conversion f.

De manière connue, la réponse d'un capteur semi-conducteur 1 dérive au cours de son utilisation, notamment à cause de l'obstruction progressive des pores de la couche d'oxyde métallique due à l'adsorption des COV 40 qui modifie la conductivité électrique U mesurée. Autrement dit, la conductivité électrique U mesurée à plusieurs mois d'intervalles dans des conditions identiques est différente, bien que la concentration réelle en COV soit identique. La concentration S1 en COV 4 déterminée est alors faussée car la fonction de conversion f ne reflète plus la relation entre la conductivité électrique U et la concentration réelle en COV 40 dans l'air du bureau 4. Il est ainsi nécessaire d'étalonner le capteur semi-conducteur 1 pour que la concentration S1 en COV 40 mesurée reste fiable et précise. L'étalonnage consiste à corriger la fonction de conversion f pour qu'elle compense la dérive du capteur 1, c'est à dire qu'elle reflète la nouvelle relation reliant la conductivité électrique U et la concentration réelle en COV 40 dans l'air du bureau 4.

L'invention concerne un procédé d'étalonnage d'un capteur semi-conducteur 1 tel que celui décrit précédemment, ou plus généralement de tout capteur chimique non sélectif comportant une fonction de conversion f à étalonner, tel qu'un capteur électrochimique ou un capteur à photoionisation, noté « capteur à étalonner 1 » par la suite à des fins de clarté.

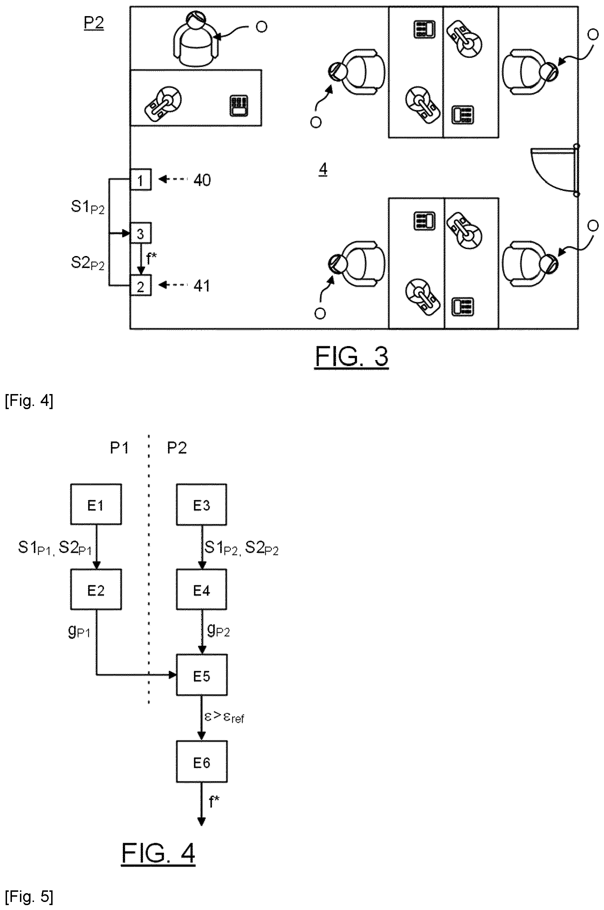

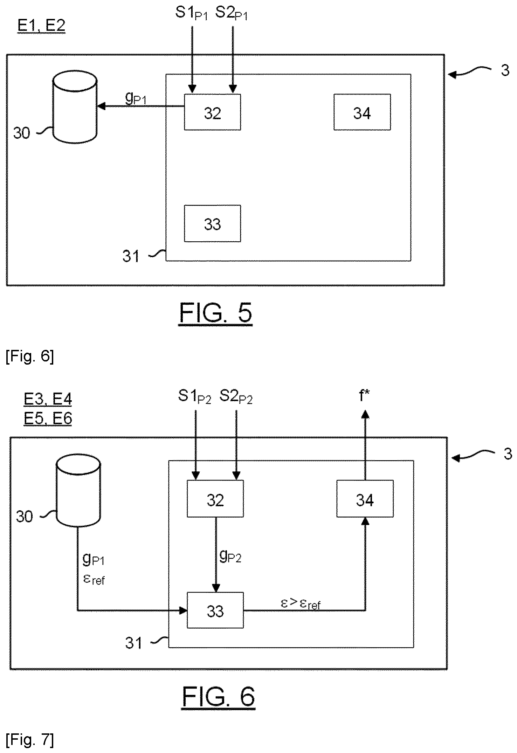

En référence aux figures 2 et 3, pour mettre en oeuvre le procédé d'étalonnage, l'invention se propose d'utiliser un système d'étalonnage comprenant le capteur à étalonner 1 et un capteur de référence 2 différent du capteur à étalonner 1 qui est mis au contact du même milieu physique que le capteur à étalonner 1, à savoir l'air intérieur d'un bureau 4 dans l'exemple des figures 2 et 3. Le système d'étalonnage comprend en outre un module de calcul 3 relié au capteur à étalonner 1 et au capteur de référence 2 et configuré pour comparer leurs mesures de manière à détecter une éventuelle dérive du capteur à étalonner 1 et calculer une fonction de conversion optimisée f *. De préférence, le module de calcul 3 se présente sous la forme d'un microprocesseur. Dans l'exemple des figures 5 et 6, le module de calcul 3 comprend une base de données 30 et un calculateur 31 avec trois unités de calcul 32, 33, 34. Il va de soi que le module de calcul 3 comprend un nombre quelconque de bases de données 30 intégrées ou non au calculateur 31 et un nombre quelconque de calculateurs 31 comprenant un nombre quelconque d'unités de calcul 32, 33, 34.

Pour que la comparaison soit pertinente, le capteur de référence 2 est choisi pour être sensible à une grandeur physico-chimique du milieu physique qui est corrélée à celle du capteur à étalonner 1. Autrement dit, le capteur de référence 2 est configuré pour mesurer un signal de référence S2 dont la variation temporelle est corrélée à celle du signal chimique S1 mesuré par le capteur à étalonner 1. De plus, le capteur de référence 2 est choisi de préférence sélectif et/ou autocalibré, de manière à que ses mesures soient précises et non sujettes à la dérive dans le temps.

Dans l'exemple des figures 2 et 3, le capteur de référence 2 se présente ainsi sous la forme d'un capteur de dioxyde de carbone 41, d'abréviation « CO2 », configuré pour déterminer la concentration S2 de CO2 41 dans l'air du bureau 4. Une telle concentration S2 de CO2 41 est corrélée à la concentration S1 de COV 40 mesurée par le capteur à étalonner 1 en ce qu'elles sont toutes deux liées au taux d'occupation O du bureau 4. En effet, le CO2 41 présent dans le bureau 4 est essentiellement émis par les occupants du bureau 4 et sa variation temporelle dépend donc du taux d'occupation O du bureau 4. Les COV 40 présents dans le bureau 4 sont quant à eux essentiellement émis par les appareils électroniques tels que les ordinateurs, les imprimantes ou encore les radiateurs, utilisés par les occupants, si bien que leur variation temporelle dépend également du taux d'occupation O du bureau 4. Par ailleurs, le capteur de référence 2 se présente de préférence sous la forme d'un capteur infrarouge non dispersif, autocalibré et sélectif pour le CO2 41.

Il va de soi qu'un autre capteur de référence 2 pourrait être choisi, l'essentiel étant qu'il soit configuré pour mesurer un signal de référence S2 corrélé au signal chimique S1 du capteur à étalonner 1, précis et non sujet à la dérive. Ainsi, dans cet exemple, le capteur de référence 2 pourrait être sensible à une autre grandeur physique que le CO2 41, telle que le dioxygène qui est essentiellement consommé par les occupants du bureau 4 et dont la quantité est donc corrélée à celle de COV 40. Le CO2 présente toutefois l'avantage de varier davantage en proportion par rapport au dioxygène, ce qui permet un étalonnage plus précis. Par ailleurs, dans cet exemple, le capteur de référence 2 pourrait être de nature différente, telle qu'un capteur électrochimique ou infrarouge non dispersif, qui doit être sélectif et autocalibré. On notera donc que le choix du capteur de référence 2 dépend du capteur à étalonner 1, et notamment du groupe de composés chimiques 40 auquel il est sensible. Dans l'exemple des figures 2 et 3, le capteur à étalonner 1 est sensible aux COV 40, mais il va de soi que le capteur à étalonner 1 pourrait être sensible à un autre groupe de composés chimiques 40.

Par ailleurs, de préférence, le capteur à étalonner 1 et le capteur de référence 2 sont installés à proximité l'un de l'autre, préférentiellement de manière adjacente, de manière à être au contact d'un même milieu physique 4 dans des conditions physico-chimiques identiques. De préférence également, le système d'étalonnage forme un module unitaire, facile à installer mais il va de soi que le module de calcul 3 pourrait être déporté pour limiter l'encombrement, ne nécessitant pas d'être au contact du milieu physique 4.

On décrit par la suite un procédé d'étalonnage mis en oeuvre par le système d'étalonnage précédemment décrit. En référence à la figure 4, le procédé comprend :
- une étape de mesure E1, pendant une période de référence P1 illustrée sur la figure 2, d'un premier signal chimique S1_{P1} au moyen du capteur à étalonner 1 et d'un premier signal de référence S2_{P1} au moyen du capteur de référence 2,
- une étape de détermination E2 d'une première fonction de régression g_{P1} définie de la manière suivante : S2_{P1} = g_{P1}(S1_{P1}),
- une étape de mesure E3, pendant une période de test P2 illustrée sur la figure 3 et postérieure à la période de référence P1, d'un deuxième signal chimique S1_{P2} au moyen du capteur à étalonner 1 et d'un deuxième signal de référence S2_{P2} au moyen du capteur de référence 2,
- une étape de détermination E4 d'une deuxième fonction de régression g_{P2} définie de la manière suivante : S2_{P2} = g_{P2}(S1_{P2}),
- une étape de calcul E5 d'un écart ε entre la première fonction de régression g_{P1} et la deuxième fonction de régression g_{P2} et
- lorsque l'écart ε est supérieur à un écart de référence ε_{ref}, une étape de détermination E6 d'une fonction de conversion optimisée f* afin que la deuxième fonction de régression g_{P2} soit sensiblement égale à la première fonction de régression g_{P1}, de manière à étalonner le capteur à étalonner 1.

De manière avantageuse, un tel procédé d'étalonnage est réalisé dans le milieu physique et ne nécessite pas de déplacer le capteur à étalonner 1 dans un ou plusieurs milieux étalons comme dans l'art antérieur, ce qui présente un gain en temps et en ressources. En outre, un tel procédé d'étalonnage peut être réalisé de manière autonome par le système d'étalonnage et en particulier le module de calcul sous forme de programme d'ordinateur, et ne nécessite donc pas d'intervention humaine comme dans l'art antérieur. La mise en oeuvre du procédé d'étalonnage dans le milieu physique présente également l'avantage d'être plus pertinente, car la fonction de conversion optimisée f* est alors spécifiquement calculée pour le milieu d'intérêt et les composés chimiques d'intérêt. La précision du procédé d'étalonnage est en outre garantie par le choix du capteur de référence 2, à savoir sensible à une grandeur physique dont la variation temporelle est corrélée à celle du capteur à étalonner. Le capteur de référence 2 est de préférence également choisi sélectif et autocalibré pour fournir des mesures précises et non sujettes à la dérive.

De préférence, le procédé d'étalonnage est mis en oeuvre pour plusieurs périodes de test P2. Plus précisément, pour chacune des périodes de test P2, une étape de mesure E3, une étape de détermination E4, une étape de calcul E5 et une étape de détermination E6 sont mises en oeuvre. De manière avantageuse, le procédé d'étalonnage est répétable aussi souvent qu'on le souhaite. Un étalonnage régulier voire périodique, par exemple tous les trois mois, peut ainsi être mis en place pour contrôler de manière périodique la potentielle dérive du capteur à étalonner 1 et la corriger. De manière avantageuse, un tel procédé d'étalonnage garantit ainsi la précision et la fiabilité des mesures du capteur à étalonner 1 au fur et à mesure de son utilisation. On notera également que peu importe le nombre de périodes de test P2, l'étape de mesure E1 et l'étape de détermination E2 ne sont mises en oeuvre qu'une seule fois, ce qui présente également un gain de temps et augmente la pertinence de l'étalonnage. En effet, la première fonction de régression g_{P1} sert de témoin et de base de comparaison pour la ou les deuxième(s) fonction(s) de régression déterminée(s) par la suite.

On décrit par la suite plus précisément chacune des étapes du procédé d'étalonnage, en ne considérant qu'une période de test P2. Les étapes sont de plus décrites dans le cadre du système d'étalonnage précédemment décrit, à savoir formé par un capteur semi-conducteur 1 de COV 40 (i.e. le capteur à étalonner), un capteur à infrarouge 2 de CO2 41 (i.e. le capteur de référence) et le module de calcul 3, tel qu'un microprocesseur.

Le procédé d'étalonnage démarre par une étape de mesure E1 simultanée de la concentration S1 en COV 40 et de la concentration S2 en CO2 41 de l'air intérieur du bureau 4 illustré sur les figures 2 et 3. En référence à la figure 7, la concentration S1 en COV 40 est mesurée par le capteur à étalonner pendant une période de référence P1 et notée « première concentration S1_{P1} en COV 40 ». La concentration S2 en CO2 41 de l'air intérieur du bureau 4 est quant à elle mesurée par le capteur de référence 2 pendant la même période de référence P1 et notée « première concentration S2_{P1} en CO2 41 ».

La période de référence P1 est de préférence choisie telle que la réponse du capteur à étalonner 1 soit réputée valide, c'est-à-dire que le capteur à étalonner 1 est réputé étalonné et ne dérive pas. Autrement dit, la première concentration S1_{P1} en COV 40 est sensiblement identique à la concentration réelle en COV 40 de l'air intérieur du bureau 4. En pratique, la période de référence P1 est choisie dans les jours suivant l'installation et/ou la configuration du capteur à étalonner 1 dans le bureau 4. De manière alternative, la période de référence P1 est choisie dans les jours suivant son étalonnage, par exemple dans un ou plusieurs milieux étalons tel que mis en oeuvre dans l'art antérieur.

Par ailleurs, toujours en référence à la figure 7, la période de référence P1 est également choisie suffisamment longue pour mesurer toute la plage de variabilité de la concentration S1 en COV 40 dans l'air intérieur du bureau 4, et notamment sa valeur maximale et sa valeur minimale. Dans l'exemple de la figure 7, la concentration S1 en COV 40 est fonction du taux d'occupation O du bureau 4 illustré sur la figure 2 et variant entre 0 occupant (taux d'occupation minimal Omin) et 5 occupants (taux d'occupation maximal Omax). De manière classique, les occupants arrivent progressivement le matin, partent déjeuner le midi et reviennent l'après-midi avant de rentrer progressivement chez eux. Le taux d'occupation est donc minimal Omin le midi, le matin avant l'arrivée du premier occupant et le soir après le départ du dernier occupant. Le taux d'occupation est à contrario maximal Omax au cours du matin et de l'après-midi. Dans l'exemple de la figure 7, la période de référence P1 est ainsi choisie égale à un jour, ce qui est suffisant pour mesurer la première concentration S1_{P1} lorsque le taux d'occupation est minimal Omin et maximal Omax. De manière générale, la période de référence P1 est de préférence comprise entre un jour et un mois, de préférence de l'ordre d'une semaine.

Toujours en référence à la figure 7, la première concentration S1_{P1} en COV 40 correspond à une pluralité de points de mesure (représentés par des triangles sur la figure 7) espacés d'un pas de mesure Δt. Dans cet exemple, le pas de mesure Δt est égal à 1h, suffisamment petit pour observer la variation temporelle de la première concentration S1_{P1} et suffisamment grand pour limiter la taille des données mesurées. De manière générale, le pas de mesure Δt est de préférence compris entre 1 min et 4h, dépendant du milieu physique et de la grandeur physique ou chimique mesurée. La première concentration S2_{P1} en CO2 41 comprend quant à elle de préférence le même nombre de points de mesure (représentés par des croix sur la figure 7) mesurés au même instant de mesure que pour la première concentration S1_{P1} en COV 40. Dans cet exemple, les points de mesure correspondent à des valeurs instantanées mais il va de soi que les points de mesure pourraient également correspondre à des valeurs moyennées sur une durée, par exemple égale au pas de mesure Δt.

En référence à la figure 5, à la fin de l'étape de mesure E1, la première concentration S1_{P1} en COV 40 et la première concentration S2_{P1} en CO2 41 sont transmises au module de calcul 3, et plus précisément dans l'exemple de la figure 5 à une première unité de calcul 32 du module de calcul 3.

En référence aux figures 4, 5 et 8, le procédé d'étalonnage se poursuit par une étape de détermination E2 d'une première fonction de régression g_{P1} définie de la manière suivante : S2_{P1} = g_{P1}(S1 _{P1}). Autrement dit, la première fonction de régression g_{P1} correspond à l'évolution de la première concentration S2_{P1} en CO2 41 en fonction de la première concentration S1_{P1} en COV 40 sur la période de référence P1. Un point de la première fonction de régression g_{P1} est donc obtenu par un point de mesure de la première concentration S1_{P1} en COV 40 et un point de mesure de la première concentration S2_{P1} en CO2 41 au même instant de mesure. Une telle première fonction de régression g_{P1} permet d'obtenir la relation de corrélation reliant la première concentration S1_{P1} en COV 40 et la première concentration S2_{P1} en CO2 41. Le capteur à étalonner 1 étant réputé valide pendant la période de référence P1, une telle première fonction de régression g_{P1} illustre la relation de corrélation réelle, précise et juste existant entre la concentration S2 en CO2 41 et la concentration réelle en COV 40, et sert donc de témoin par la suite pour évaluer la dérive du capteur à étalonner 1 et corriger la fonction de conversion f. De préférence, la première fonction de régression g_{P1} est polynomiale, de préférence affine, préférentiellement linéaire de manière à être simple à déterminer et à utiliser par la suite.

En référence à la figure 5, à la fin de l'étape de détermination E2, la première fonction de régression g_{P1} est stockée dans le module de calcul 3 et plus précisément dans la base de données 30.

En référence aux figures 6 et 7, le procédé d'étalonnage se poursuit par une étape de mesure E3 similaire à l'étape de mesure E1 si ce n'est qu'elle est mise en oeuvre lors d'une période de test P2 ultérieure à la période de référence P1. La concentration S1 en COV 40 et la concentration S2 en CO2 41 mesurées lors la période de test P2 sont respectivement notées « deuxième concentration S1_{P2} en COV 40 » et « deuxième concentration S2_{P2} en CO2 41 ».

De préférence, la période de test P2 est mise en oeuvre lorsque le capteur à étalonner 1 est susceptible d'avoir dérivé, par exemple entre 1 mois et 12mois après la période de référence P1 ou après son dernier étalonnage. Il va cependant de soi que la période de test P2 pourrait être mise en oeuvre plus tôt pour vérifier le bon fonctionnement du capteur à étalonner 1 ou suite à une anomalie observée à titre d'exemples. De même que la période de référence P1, la période de test P2 est de préférence choisie suffisamment longue pour mesurer toute la plage de variabilité de la concentration S1 en COV 40 dans l'air intérieur du bureau 4, et notamment sa valeur maximale et sa valeur minimale. Préférentiellement, la période de test P2 est choisie de durée identique à la période de référence P1 et avec le même nombre de points de mesure.

En référence à la figure 6, de même qu'à la fin de l'étape de mesure E1, la deuxième concentration S1_{P2} en COV 40 et la deuxième concentration S2_{P2} en CO2 41 sont transmises au module de calcul 3, et plus précisément dans l'exemple de la figure 6 à la première unité de calcul 32.

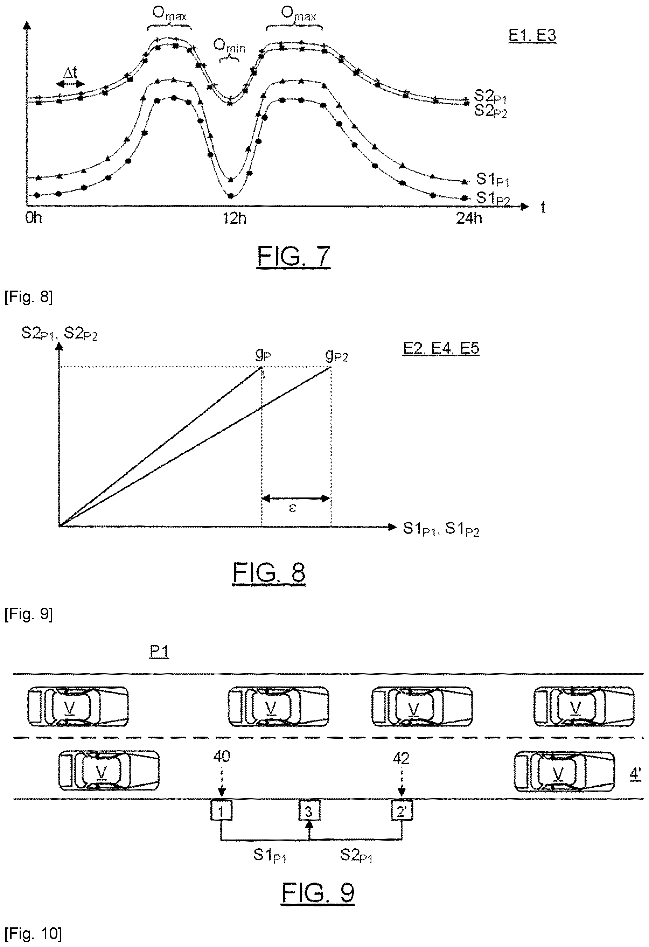

En référence aux figures 6 et 8, le procédé d'étalonnage comprend ensuite une étape de détermination E4 d'une deuxième fonction de régression g_{P2} définie de la manière suivante : S2_{P2} = g_{P2}(S1_{P2}), de manière similaire à l'étape de détermination E2. De même que la première fonction de régression g_{P1}, la deuxième fonction de régression g_{P2} est de préférence polynomiale, de préférence affine, préférentiellement linéaire de manière à être simple à déterminer et à utiliser par la suite.

Toujours en référence aux figures 6 et 8, au cours d'une étape de calcul E5, une deuxième unité de calcul 33 du module de calcul 3 détermine l'écart ε entre la première fonction de régression g_{P1} transmise par la base de données 30 et la deuxième fonction de régression g_{P2} transmise par la première unité de calcul 32.

Dans l'exemple de la figure 8, l'écart ε déterminé correspond à la différence maximale observée entre la première concentration S1_{P1} et la deuxième concentration S1_{P2} en COV 40 pour une concentration S2 donnée et s'exprime selon la relation suivante : ε = Max (|S1_{P1}-S1_{P2}|) / S1_{P1} tel que S2_{P1}=S2_{P2}. Il va de soi que l'écart ε entre les deux fonctions de régression g_{P1}, g_{P2} pourrait être déterminé autrement, par exemple en chaque point de mesure plutôt qu'au point de mesure où l'écart ε est maximal.

De manière avantageuse, un tel écart ε est représentatif de l'éventuelle dérive du capteur à étalonner 1. En effet, parmi les quatre concentrations mesurées S1_{P1}, S1_{P2}, S2_{P1}, S2_{P2}, seule la deuxième concentration S1_{P2} en COV 40 n'est pas réputée valide, si bien que l'écart ε observé n'est dû qu'à la dérive du capteur à étalonner 1 entre la période de référence P1 et la période de test P2. On note ici la nécessité de la présence d'un capteur de référence 2 qui sert à connaître de manière précise et fiable la composition de l'air intérieur du bureau 4 qui est à priori différente pour la période de référence P1 et la période de test P2. Une comparaison entre la première concentration S1_{P1} et la deuxième concentration S2_{P1} en COV 40 ne serait en effet pas pertinente car les mesures ne seraient pas effectuées dans un milieu physique de composition chimique identique. En effet, à titre d'exemple, un des occupants pourrait être absent lors de la période de test P2, réduisant le taux d'occupation maximal Omax et ainsi la quantité de COV 40 dans l'air intérieur du bureau 4. On note donc également la nécessité d'avoir un capteur de référence 2 réputé valide à tout instant, car une dérive du capteur de référence 2 rendrait l'écart ε non représentatif de la dérive du capteur à étalonner 1.

En référence à la figure 6, le procédé d'étalonnage se termine par une étape de détermination E6 au cours de laquelle, si l'écart ε est supérieur à un écart minimal ε_{ref}, une troisième unité de calcul 34 du module de calcul 3 détermine une fonction de conversion optimisée f* pour le capteur à étalonner 1, de manière à corriger la dérive. L'écart minimal ε_{ref} correspond au seuil au-dessus duquel on considère que la dérive est non négligeable. De préférence, l'écart minimal ε_{ref} provient de la base de données 30 et est compris entre 0,01 et 0,1, de préférence de l'ordre de 0,05, ce qui permet d'éviter toute dérive importante tout en évitant de devoir réaliser une micro-correction peu pertinente. En pratique, la valeur de l'écart minimal ε_{ref} est ajustée par retour d'expérience.

Dans l'exemple de la figure 6, l'étape de détermination E6 est mise en oeuvre en calculant la fonction de conversion optimisée f* de la manière suivante : g_{P2}(f*(U_{P2})) = g_{P1}(S1_{P1}), en notant U_{P2} la conductivité électrique de la couche d'oxyde métallique du capteur à étalonner 1 mesurée lors de la période de test P2 et qui est reliée à la deuxième concentration S1_{P2} en COV 40 par la relation : S1_{P2} = f(U_{P2}). Autrement dit, la fonction de conversion optimisée f* est calculée en cherchant l'égalité g_{P2} = g_{P1}, c'est-à-dire que les deux fonctions de régression g_{P1}, g_{P2} correspondent à une fonction polynomiale de mêmes coefficients.

Pour résumer, le procédé d'étalonnage selon l'invention permet de déterminer une fonction de conversion optimisée f* pour le capteur à étalonner 1 à partir de l'écart ε entre une première fonction de régression g_{P1} pour une période de référence P1 où le capteur à étalonner 1 est réputé valide et une deuxième fonction de régression g_{P2} pour une période de test P2 où le capteur à étalonner 1 a potentiellement dérivé. Les deux fonctions de régression g_{P1}, g_{P2} sont calculées en mettant en relation les grandeurs physiques mesurées par le capteur à étalonner 1 et un capteur de référence 2, réputé valide à tout instant, dans un même milieu physique 4. Le capteur de référence 2 permet ainsi de connaître la composition du milieu physique 4 à tout instant et donc d'évaluer la dérive du capteur à étalonner 1.

Par ailleurs, dans l'exemple des figures 2 et 3, le procédé d'étalonnage est réalisé dans l'air intérieur d'un bureau 4 au moyen d'un système d'étalonnage comprenant un capteur semi-conducteur 1 de COV 40, un capteur à infrarouge 2 de CO2 41 et un microprocesseur 3, mais il va de soi que le procédé d'étalonnage peut être réalisé dans tout milieu physique et au moyen d'un capteur à étalonner 1 se présentant sous la forme d'un capteur chimique non sélectif quelconque, le capteur de référence 2 étant choisi en fonction du capteur à étalonner 1 et du milieu physique 4 et le module de calcul 3 comprenant au moins une base de données 30 et un calculateur 31.

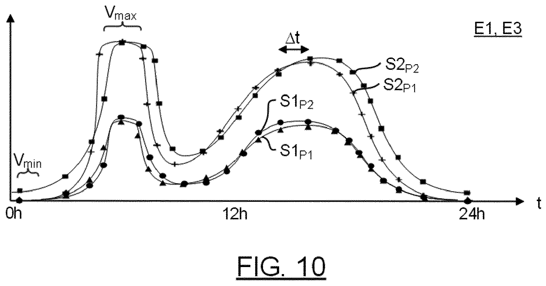

Ainsi, dans l'exemple des figures 9 et 10 illustrant un autre mode et une autre forme de réalisation de l'invention, le procédé d'étalonnage est mis en oeuvre à proximité d'un axe routier 5 où circulent des véhicules V. Le capteur à étalonner 1 se présente de même que précédemment sous la forme d'un capteur semi-conducteur de COV 40. Le capteur de référence 2 se présente quant à lui sous la forme d'un capteur d'intensité sonore 42 de l'axe routier 5. Un tel capteur d'intensité sonore 42 est avantageusement autocalibré et réalise des mesures précises ce qui en fait un capteur de référence adapté. De plus, l'intensité sonore 42 est fonction du trafic de véhicules V sur l'axe routier 5 de même que la concentration en COV 40 émise à proximité de l'axe routier 5, si bien que les grandeurs physiques mesurées par le capteur à étalonner 1 et le capteur de référence 2 sont corrélées. De manière classique, en référence à la figure 10, le trafic de véhicules V connaît un pic le matin correspondant au déplacement pour aller au travail et un pic plus étendu l'après-midi et le soir correspondant au déplacement pour rentrer du travail. Le trafic de véhicules V est ainsi maximal Vmax le matin et minimal Vmin très tôt le matin. Dans l'exemple de la figure 10, la période de référence P1 est ainsi choisie égale à un jour de même que dans l'exemple des figures 2 et 3, car une telle période de référence P1 est suffisante pour mesurer la première concentration S1_{P1} lorsque le trafic de véhicules V est minimal Vmin et maximal Vmax.

Il a été décrit précédemment un système d'étalonnage comprenant un unique capteur à étalonner 1 et un unique capteur de référence 2, mais il va de soi que le système d'étalonnage pourrait comprendre plusieurs capteurs à étalonner 1 configurés chacun pour être étalonnés par un capteur de référence 2. De préférence, un tel système d'étalonnage comprend un unique capteur de référence 2 permettant l'étalonnage de chaque capteur à étalonner 1 pour limiter l'encombrement. Ainsi, à titre d'exemple, en reprenant la forme de réalisation de l'invention de la figure 10, le capteur à étalonner 1 se présentant sous la forme d'un capteur semi-conducteur de COV 40 pourrait être remplacé par une barrette de capteurs à étalonner 1 comprenant un capteur semi-conducteur et un capteur électrochimique de COV 40 ainsi qu'un capteur de monoxyde de carbone et d'oxyde d'azote. Une telle barrette de capteurs présente l'avantage de mesurer de manière plus précise la qualité de l'air à proximité de l'axe routier qu'un capteur semi-conducteur de COV 40 seul. Le capteur de référence 2 reste inchangé et se présente sous la forme d'un capteur d'intensité sonore 42, l'intensité sonore 42 émises par la circulation étant reliée à la quantité de COV produite, mais également à celles de monoxyde de carbone et d'oxyde d'azote.

Pour un tel système d'étalonnage, le procédé d'étalonnage est mis en oeuvre en mesurant un premier signal chimique S1P1 et un deuxième signal chimique S1P2 à partir de chaque capteur à étalonner 1. Une première fonction de régression g_{P1} et une deuxième fonction de régression g_{P2} sont ensuite calculées pour chaque capteur à étalonner 1. Un écart ε est ensuite déterminé pour chaque capteur à étalonner 1 afin de déterminer pour chacun l'étalonnage requis de manière indépendante.

## Revendications

1. Procédé d'étalonnage d'au moins un capteur chimique non sélectif, dit « capteur à étalonner (1) », au moyen d'au moins un capteur de référence (2, 2'), ledit capteur à étalonner (1) et ledit capteur de référence (2, 2') étant au contact d'un même milieu chimique (4, 4'), ledit capteur à étalonner (1) étant configuré pour déterminer un signal chimique (S1) de la quantité d'un groupe de composés chimiques (40) dans ledit milieu chimique (4, 4'), ledit capteur de référence (2, 2') étant configuré pour déterminer un signal de référence (S2) dont la variation temporelle est corrélée à celle dudit signal chimique (S1), ledit capteur à étalonner (1) comprenant :
- un élément de mesure (10) d'un signal électrique (U), qui est fonction de la quantité dudit groupe de composés chimiques (40) dans ledit milieu chimique (4, 4'), et
- un élément de calcul (11) dudit signal chimique (S1) à partir d'une fonction de conversion (f) propre audit capteur à étalonner (1) et configurée pour associer audit signal électrique (U) mesuré le signal chimique (S1) correspondant suivant la relation suivante : S1 = f(U),
- ledit procédé étant **caractérisé par le fait qu'**il comprend :
- une étape de mesure (E1), pendant une période de référence (P1), d'un premier signal chimique (S1_{P1}) au moyen du capteur à étalonner (1) et d'un premier signal de référence (S2_{P1}) au moyen du capteur de référence (2, 2'),
- une étape de détermination (E2) d'une première fonction de régression (g_{P1}) définie de la manière suivante : S2_{P1} = g_{P1}(S1_{P1}),
- au moins une étape de mesure (E3), pendant une période de test (P2) postérieure à la période de référence (P1), d'un deuxième signal chimique (S1_{P2}) au moyen du capteur à étalonner (1) et d'un deuxième signal de référence (S2_{P2}) au moyen du capteur de référence (2, 2'),
- au moins une étape de détermination (E4) d'une deuxième fonction de régression (g_{P2}) définie de la manière suivante : S2_{P2} = g_{P2}(S1_{P2}),
- au moins une étape de calcul (E5) d'un écart (ε) entre la première fonction de régression (g_{P1}) et la deuxième fonction de régression (g_{P2}) et
- lorsque l'écart (ε) est supérieur à un écart de référence (ε_{ref}), au moins une étape de détermination (E6) d'une fonction de conversion optimisée (f*) afin que la deuxième fonction de régression (g_{P2}) soit sensiblement égale à la première fonction de régression (g_{P1}), de manière à étalonner le capteur à étalonner (1).

2. Procédé d'étalonnage selon la revendication 1, mis en oeuvre pour une pluralité de périodes de test (P2) postérieures à la période de référence (P1), ledit procédé comprenant pour chaque période de test (P2) :
- une étape de mesure (E3), pendant la période de test (P2), d'un deuxième signal chimique (S1_{P2}) au moyen du capteur à étalonner (1) et d'un deuxième signal de référence (S2_{P2}) au moyen du capteur de référence (2),
- une étape de détermination (E4) d'une deuxième fonction de régression (g_{P2}) définie de la manière suivante : S2_{P2} = g_{P2}(S1_{P2}),
- une étape de calcul (E5) d'un écart (ε) entre la première fonction de régression (g_{P1}) et la deuxième fonction de régression (g_{P2}) et
- lorsque l'écart (ε) est supérieur à un écart de référence (ε_{ref}), une étape de détermination (E6) d'une fonction de conversion optimisée (f*) afin que la deuxième fonction de régression (g_{P2}) soit sensiblement égale à la première fonction de régression (g_{P1}), de manière à étalonner le capteur à étalonner (1).

3. Procédé d'étalonnage selon l'une des revendications 1 et 2, dans lequel le capteur à étalonner (1) se présente sous la forme d'un capteur de COV.

4. Procédé d'étalonnage selon l'une des revendications 1 à 3, dans lequel le capteur de référence (2) se présente sous la forme d'un capteur de dioxyde de carbone (41).

5. Procédé d'étalonnage selon l'une des revendications 1 à 3, dans lequel le capteur de référence (2') se présente sous la forme d'un capteur d'intensité sonore (42).

6. Procédé d'étalonnage selon l'une des revendications 1 à 5, dans lequel la période de référence (P1) est comprise entre un jour et un mois, de préférence de l'ordre d'une semaine.

7. Procédé d'étalonnage selon l'une des revendications 1 à 6, dans lequel l'écart (ε) vérifie la relation suivante : ε = Max (|S1_{P1}-S1_{P2}|) / S1_{P1} tel que S2_{P1} = S2_{P2}.

8. Procédé d'étalonnage selon l'une des revendications 1 à 7, dans lequel, le deuxième signal chimique (S1_{P2}) étant déterminé à partir d'un deuxième signal électrique (U_{P2}), la fonction de conversion optimisée (f*) vérifie la relation suivante : g_{P2}(f*(U_{P2})) = g_{P1}(S1_{P1}).

9. Système d'étalonnage pour la mise en oeuvre du procédé d'étalonnage selon l'une des revendications 1 à 8, ledit système comprenant au moins un capteur chimique non sélectif, dit « capteur à étalonner (1) » et au moins un capteur de référence (2, 2') configurés pour être au contact d'un même milieu chimique (4, 4') et un module de calcul (3) relié au capteur à étalonner (1) et au capteur de référence (2), ledit capteur à étalonner (1) étant configuré pour déterminer un signal chimique (S1) de la quantité d'un groupe de composés chimiques (40) dans ledit milieu chimique (4, 4'), ledit signal chimique (S1) comprenant un premier signal chimique (S1_{P1}) pendant une période de référence (P1) et un deuxième signal chimique (S1_{P2}) pendant une période de test (P2), ledit capteur de référence (2, 2') étant configuré pour déterminer un signal de référence (S2) dont la variation temporelle est corrélée à celle dudit signal chimique (S1), ledit signal de référence (S2) comprenant un premier signal de référence (S2_{P1}) pendant ladite période de référence (P1) et un deuxième signal de référence (S2_{P2}) pendant ladite période de test (P2), ledit capteur à étalonner (1) comprenant :
- un élément de mesure (10) d'un signal électrique (U), qui est fonction de la quantité dudit groupe de composés chimiques (40) dans ledit milieu chimique (4, 4'), et
- un élément de calcul (11) dudit signal chimique (S1) à partir d'une fonction de conversion (f) propre audit capteur à étalonner (1) et configurée pour associer audit signal électrique (U) mesuré le signal chimique (S1) correspondant suivant la relation suivante : S1 = f(U),
- ledit module de calcul (3) étant configuré pour :
- déterminer une première fonction de régression (g_{P1}) et une deuxième fonction de régression (g_{P2}) définies de la manière suivante : S2_{P1} = g_{P1}(S1_{P1}) et S2_{P2}=g_{P2}(S1 _{P2})
- calculer un écart (ε) entre la première fonction de régression (g_{P1}) et la deuxième fonction de régression (g_{P2}) et
- lorsque l'écart (ε) est supérieur à un écart de référence (ε_{ref}), déterminer une fonction de conversion optimisée (f*) afin que la deuxième fonction de régression (g_{P2}) soit sensiblement égale à la première fonction de régression (g_{P1}), de manière à étalonner le capteur à étalonner (1).

10. Programme d'ordinateur comprenant des instructions configurées pour être exécutées sur un ordinateur de sorte que le système d'étalonnage selon la revendication 9 met en oeuvre :
- une étape de mesure (E1), pendant une période de référence (P1), d'un premier signal chimique (S1_{P1}) au moyen du capteur à étalonner (1) et d'un premier signal de référence (S2_{P1}) au moyen du capteur de référence (2, 2'),
- une étape de détermination (E2) d'une première fonction de régression (g_{P1}) définie de la manière suivante : S2_{P1} = g_{P1}(S1_{P1}),
- au moins une étape de mesure (E3), pendant une période de test (P2) postérieure à la période de référence (P1), d'un deuxième signal chimique (S1_{P2}) au moyen du capteur à étalonner (1) et d'un deuxième signal de référence (S2_{P2}) au moyen du capteur de référence (2, 2'),
- au moins une étape de détermination (E4) d'une deuxième fonction de régression (g_{P2}) définie de la manière suivante : S2_{P2} = g_{P2}(S1_{P2}),
- au moins une étape de calcul (E5) d'un écart (ε) entre la première fonction de régression (g_{P1}) et la deuxième fonction de régression (g_{P2}) et
- lorsque l'écart (ε) est supérieur à un écart de référence (ε_{ref}), au moins une étape de détermination (E6) d'une fonction de conversion optimisée (f*) afin que la deuxième fonction de régression (g_{P2}) soit sensiblement égale à la première fonction de régression (g_{P1}), de manière à étalonner le capteur à étalonner (1).

## Patentansprüche

1. Verfahren für die Kalibrierung mindestens eines chemischen nicht-selektiven Sensors, bezeichnet als "zu kalibrierender Sensor (1)", mittels mindestens eines Referenzsensors (2, 2'), wobei der zu kalibrierende Sensor (1) und der Referenzsensor (2, 2') im Kontakt mit einem selben chemischen Medium (4, 4') sind, wobei der zu kalibrierende Sensor (1) dazu ausgelegt ist, ein chemisches Signal (S1) der Menge einer Gruppe chemischer Verbindungen (40) in dem chemischen Medium (4, 4') zu bestimmen, wobei der Referenzsensor (2, 2') dazu ausgelegt ist, ein Referenzsignal (S2) zu bestimmen, dessen zeitliche Schwankung mit der des chemischen Signals (S1) korreliert ist, wobei der zu kalibrierende Sensor (1) umfasst:
- ein Messelement (10) eines elektrischen Signals (U), das von der Menge der Gruppe chemischer Verbindungen (40) in dem chemischen Medium (4, 4') abhängt, und
- ein Berechnungselement (11) des chemischen Signals (S1) ausgehend von einer spezifischen Konversionsfunktion (f) des zu kalibrierenden Sensors (1) und die dazu ausgelegt ist, dem gemessenen elektrischen Signal (U) das entsprechende chemische Signal (S1) zuzuordnen gemäß der folgenden Formel: S1 = f(U),
wobei das Verfahren **dadurch gekennzeichnet ist, dass** es umfasst:
- einen Schritt des Messens (E1) während eines Referenzzeitraums (P1) eines ersten chemischen Signals (S1_{P1}) mittels des zu kalibrierenden Sensors (1) und eines ersten Referenzsignals (S2_{P1}) mittels des Referenzsensors (2, 2'),
- einen Schritt des Bestimmens (E2) einer ersten Regressionsfunktion (g_{P1}), die folgendermaßen definiert ist: S2_{P1} = g_{P1}(S1_{P1}),
- mindestens einen Schritt des Messens (E3) während eines Testzeitraums (P2) nach dem Referenzzeitraum (P1) eines zweiten chemischen Signals (S1_{P2}) mittels des zu kalibrierenden Sensors (1) und eines zweiten Referenzsignals (S2_{P2}) mittels des Referenzsensors (2, 2'),
- mindestens einen Schritt des Bestimmens (E4) einer zweiten Regressionsfunktion (g_{P2}), die folgendermaßen definiert ist: S2_{P2} = g_{P2}(S1_{P2}),
- mindestens einen Schritt des Berechnens (E5) einer Abweichung (ε) zwischen der ersten Regressionsfunktion (g_{P1}) und der zweiten Regressionsfunktion (g_{P2}) und
- wenn die Abweichung (ε) größer als eine Referenzabweichung (ε_{ref}) ist, mindestens einen Schritt des Bestimmens (E6) einer optimierten Konversionsfunktion (f*), damit die zweite Regressionsfunktion (g_{P2}) etwa gleich der ersten Regressionsfunktion (g_{P1}) ist, so dass der zu kalibrierende Sensor (1) kalibriert wird.

2. Kalibrierungsverfahren nach Anspruch 1, das für eine Vielzahl von Testzeiträumen (P2) nach dem Referenzzeitraum (P1) durchgeführt wird, wobei das Verfahren für jeden Testzeitraum (P2) umfasst:
- einen Schritt des Messens (E3) während des Testzeitraums (P2) eines zweiten chemischen Signals (S1_{P2}) mittels des zu kalibrierenden Sensors (1) und eines zweiten Referenzsignals (S2_{P2}) mittels des Referenzsensors (2),
- einen Schritt des Bestimmens (E4) einer zweiten Regressionsfunktion (g_{P2}), die folgendermaßen definiert ist: S2_{P2} = g_{P2}(S1_{P2}),
- einen Schritt des Berechnens (E5) einer Abweichung (ε) zwischen der ersten Regressionsfunktion (g_{P1}) und der zweiten Regressionsfunktion (g_{P2}) und
- wenn die Abweichung (ε) größer als eine Referenzabweichung (ε_{ref}) ist, einen Schritt des Bestimmens (E6) einer optimierten Konversionsfunktion (f*), damit die zweite Regressionsfunktion (g_{P2}) etwa gleich der ersten Regressionsfunktion (g_{P1}) ist, so dass der zu kalibrierende Sensor (1) kalibriert wird.

3. Kalibrierungsverfahren nach einem der Ansprüche 1 und 2, wobei der zu kalibrierende Sensor (1) in Form eines COV-Sensors vorliegt.

4. Kalibrierungsverfahren nach einem der Ansprüche 1 bis 3, wobei der Referenzsensor (2) in Form eines Kohlendioxidsensors (41) vorliegt.

5. Kalibrierungsverfahren nach einem der Ansprüche 1 bis 3, wobei der Referenzsensor (2') in Form eines Tonstärkesensors (42) vorliegt.

6. Kalibrierungsverfahren nach einem der Ansprüche 1 bis 5, wobei der Referenzzeitraum (P1) zwischen einem Tag und einem Monat, vorzugsweise in der Größenordnung von einer Woche liegt.

7. Kalibrierungsverfahren nach einem der Ansprüche 1 bis 6, wobei die Abweichung (ε) die folgende Gleichung erfüllt: ε = Max (|S1_{P1}-S1_{P2}|) / S1_{P1} so dass S2_{P1} = S2_{P2}.

8. Kalibrierungsverfahren nach einem der Ansprüche 1 bis 7, wobei, wobei das zweite chemische Signal (S1_{P2}) ausgehend von einem zweiten elektrischen Signal (U_{P2}) bestimmt wird, die optimierte Konversionsfunktion (f*) die folgende Gleichung erfüllt: g_{P2}(f*(U_{P2})) = g_{P1}(S1_{P1}).

9. Kalibrierungssystem für die Durchführung des Kalibrierungsverfahrens nach einem der Ansprüche 1 bis 8, wobei das System mindestens einen chemischen nicht-selektiven Sensor, bezeichnet als "zu kalibrierender Sensor (1)", und mindestens einen Referenzsensor (2, 2') umfasst, die dazu ausgelegt sind, mit einem selben chemischen Medium (4, 4') im Kontakt zu sein und ein Berechnungsmodul (3), das mit dem zu kalibrierenden Sensor (1) und dem Referenzsensor (2) verbunden ist, wobei der zu kalibrierende Sensor (1) dazu ausgelegt ist, ein chemisches Signal (S1) der Menge einer Gruppe chemischer Verbindungen (40) in dem chemischen Medium (4, 4') zu bestimmen, wobei das chemische Signal (S1) erstes chemisches Signal (S1_{P1}) während eines Referenzzeitraums (P1) und ein zweiten chemisches Signal (S1_{P2}) während eines Testzeitraums (P2) umfasst, wobei der Referenzsensor (2, 2') dazu ausgelegt ist, ein Referenzsignal (S2) zu bestimmen, dessen zeitliche Schwankung mit der des chemischen Signals (S1) korreliert ist, wobei das Referenzsignal (S2) ein erstes Referenzsignal (S2_{P1}) während des Referenzzeitraums (P1) und ein zweites Referenzsignal (S2_{P2}) während des Testzeitraums (P2) umfasst, wobei der zu kalibrierende Sensor (1) umfasst:
- ein Messelement (10) eines elektrischen Signals (U), das von der Menge der Gruppe chemischer Verbindungen (40) in dem chemischen Medium (4, 4') abhängt, und
- ein Berechnungselement (11) des chemischen Signals (S1) ausgehend von einer spezifischen Konversionsfunktion (f) des zu kalibrierenden Sensors (1) und die dazu ausgelegt ist, dem gemessenen elektrischen Signal (U) das entsprechende chemische Signal (S1) zuzuordnen gemäß der folgenden Formel: S1 = f(U),
wobei das Berechnungsmodul (3) ausgelegt ist, um:
- eine erste Regressionsfunktion (g_{P1}) und eine zweite Regressionsfunktion (g_{P2}) zu bestimmen, die folgendermaßen definiert sind: S2_{P1} = g_{P1}(S1_{P1}) und S2_{P2} = g_{P2}(S1_{P2}),
- eine Abweichung (ε) zwischen der ersten Regressionsfunktion (g_{P1}) und der zweiten Regressionsfunktion (g_{P2}) zu berechnen und
- wenn die Abweichung (ε) größer als eine Referenzabweichung (ε_{ref}) ist, eine optimierte Konversionsfunktion (f*) zu bestimmen, damit die zweite Regressionsfunktion (g_{P2}) etwa gleich der ersten Regressionsfunktion (g_{P1}) ist, so dass der zu kalibrierende Sensor (1) kalibriert wird.

10. Rechnerprogramm, das Befehle umfasst, die dazu ausgelegt sind, auf einem Rechner ausgeführt zu werden, so dass das Kalibrierungssystem nach Anspruch 9 durchführt:
- einen Schritt des Messens (E1) während eines Referenzzeitraums (P1) eines ersten chemischen Signals (S1_{P1}) mittels des zu kalibrierenden Sensors (1) und eines ersten Referenzsignals (S2_{P1}) mittels des Referenzsensors (2, 2'),
- einen Schritt des Bestimmens (E2) einer ersten Regressionsfunktion (g_{P1}), die folgendermaßen definiert ist: S2_{P1} = g_{P1}(S1_{P1}),
- mindestens einen Schritt des Messens (E3) während eines Testzeitraums (P2) nach dem Referenzzeitraum (P1) eines zweiten chemischen Signals (S1_{P2}) mittels des zu kalibrierenden Sensors (1) und eines zweiten Referenzsignals (S2_{P2}) mittels des Referenzsensors (2, 2'),
- mindestens einen Schritt des Bestimmens (E4) einer zweiten Regressionsfunktion (g_{P2}), die folgendermaßen definiert ist: S2_{P2} = g_{P2}(S1_{P2}),
- mindestens einen Schritt des Berechnens (E5) einer Abweichung (ε) zwischen der ersten Regressionsfunktion (g_{P1}) und der zweiten Regressionsfunktion (g_{P2}) und
- wenn die Abweichung (ε) größer als eine Referenzabweichung (ε_{ref}) ist, mindestens einen Schritt des Bestimmens (E6) einer optimierten Konversionsfunktion (f*), damit die zweite Regressionsfunktion (g_{P2}) etwa gleich der ersten Regressionsfunktion (g_{P1}) ist, so dass der zu kalibrierende Sensor (1) kalibriert wird.

## Claims

1. Method for calibrating at least one non-selective chemical sensor, referred to as "sensor to be calibrated (1)", by means of at least one reference sensor (2, 2'), said sensor to be calibrated (1) and said reference sensor (2, 2') being in contact with a same chemical medium (4, 4'), said sensor to be calibrated (1) being configured to determine a chemical signal (S1) of the quantity of a group of chemical compounds (40) in said chemical medium (4, 4'), said reference sensor (2, 2') being configured to determine a reference signal (S2) whose temporal variation is correlated with that of said chemical signal (S1), said sensor to be calibrated (1) comprising:
- a measuring element (10) of an electrical signal (U), which is a function of the quantity of said group of chemical compounds (40) in said chemical medium (4, 4'), and
- a calculation element (11) of said chemical signal (S1) from a conversion function (f) specific to said sensor to be calibrated (1) and configured to associate said measured electrical signal (U) with the corresponding chemical signal (S1) according to the following relation: S1 = f(U),
- said method being **characterised by the fact** that it comprises:
- a step of measuring (E1), during a reference period (P1), a first chemical signal (S1_{P1}) by means of the sensor to be calibrated (1) and a first reference signal (S2_{P1}) by means of the reference sensor (2, 2'),
- a step of determining (E2) a first regression function (g_{P1}) defined as follows: S2_{P1} = g_{P1}(S1_{P1}),
- at least one step of measuring (E3), during a test period (P2) after the reference period (P1), a second chemical signal (S1_{P2}) by means of the sensor to be calibrated (1) and a second reference signal (S2_{P2}) by means of the reference sensor (2, 2'),
- at least one step of determining (E4) a second regression function (g_{P2}) defined as follows: S2_{P2} = g_{P2}(S1_{P2}),
- at least one step of calculating (E5) a difference (ε) between the first regression function (g_{P1}) and the second regression function (g_{P2}) and
- when the difference (ε) is greater than a reference difference (ε_{ref}), at least one step of determining (E6) an optimised conversion function (f*) so that the second regression function (g_{P2}) is substantially equal to the first regression function (g_{P1}), so as to calibrate the sensor to be calibrated (1).

2. Method for calibrating according to claim 1, implemented for a plurality of test periods (P2) after the reference period (P1), said method comprising for each test period (P2):
- a step of measuring (E3), during the test period (P2), a second chemical signal (S1_{P2}) by means of the sensor to be calibrated (1) and a second reference signal (S2_{P2}) by means of the reference sensor (2),
- a step of determining (E4) a second regression function (g_{P2}) defined as follows: S2_{P2} = g_{P2}(S1_{P2}),
- a step of calculating (E5) a difference (ε) between the first regression function (g_{P1}) and the second regression function (g_{P2}) and
- when the difference (ε) is greater than a reference difference (ε_{ref}), a step of determining (E6) an optimised conversion function (f*) so that the second regression function (g_{P2}) is substantially equal to the first regression function (g_{P1}), so as to calibrate the sensor to be calibrated (1).

3. Method for calibrating according to one of claims 1 and 2, wherein the sensor to be calibrated (1) is in the form of a VOC sensor.

4. Method for calibrating according to one of claims 1 to 3, wherein the reference sensor (2) is in the form of a carbon dioxide sensor (41).

5. Method for calibrating according to one of claims 1 to 3, wherein the reference sensor (2') is in the form of a sound intensity sensor (42).

6. Method for calibrating according to one of claims 1 to 5, wherein the reference period (P1) is between one day and one month, preferably about one week.

7. Method for calibrating according to one of claims 1 to 6, wherein the difference (ε) verifies the following relationship: ε= Max (|S1_{P1}-S1_{P2}|) / S1_{P1} such that S2_{P1} = S2_{P2}.

8. Method for calibrating according to one of claims 1 to 7, wherein, the second chemical signal (S1_{P2}) being determined from a second electrical signal (U_{P2}), the optimised conversion function (f*) verifies the following relationship: g_{P2}(f*(U_{P2})) = g_{P1}(S1_{P1}).

9. System for calibrating for implementing the method for calibrating according to any one of claims 1 to 8, said system comprising at least one non-selective chemical sensor, referred to as "sensor to be calibrated (1)" and at least one reference sensor (2, 2') configured to be in contact with a same chemical medium (4, 4') and a calculation module (3) connected to the sensor to be calibrated (1) and to the reference sensor (2), said sensor to be calibrated (1) being configured to determine a chemical signal (S1) of the quantity of a group of chemical compounds (40) in said chemical medium (4, 4'), said chemical signal (S1) comprising a first chemical signal (S1_{P1}) during a reference period (P1) and a second chemical signal (S1_{P2}) during a test period (P2), said reference sensor (2, 2') being configured to determine a reference signal (S2) the time variation of which is correlated with that of said chemical signal (S1), said reference signal (S2) comprising a first reference signal (S2_{P1}) during said reference period (P1) and a second reference signal (S2_{P2}) during said test period (P2), said sensor to be calibrated (1) comprising:
- a measuring element (10) of an electrical signal (U), which is a function of the quantity of said group of chemical compounds (40) in said chemical medium (4, 4'), and
- a calculation element (11) of said chemical signal (S1) from a conversion function (f) specific to said sensor to be calibrated (1) and configured to associate said measured electrical signal (U) with the corresponding chemical signal (S1) according to the following relation: S1 = f(U),
- said calculation module (3) being configured to:
- determine a first regression function (g_{P1}) and a second regression function (g_{P2}) defined as follows: S2_{P1} = g_{P1}(S1_{P1}) and S2_{P2}=g_{P2}(S1_{P2})
- calculate a difference (ε) between the first regression function (g_{P1}) and the second regression function (g_{P2}) and
- when the difference (ε) is greater than a reference difference (ε_{ref}), determine an optimised conversion function (f*) so that the second regression function (g_{P2}) is substantially equal to the first regression function (g_{P1}), so as to calibrate the sensor to be calibrated (1).

10. Computer program comprising instructions configured to be executed on a computer so that the system for calibrating according to claim 9 implements:
- a step of measuring (E1), during a reference period (P1), a first chemical signal (S1_{P1}) by means of the sensor to be calibrated (1) and a first reference signal (S2_{P1}) by means of the reference sensor (2, 2'),
- a step of determining (E2) a first regression function (g_{P1}) defined as follows: S2_{P1} = g_{P1}(S1_{P1}),
- at least one step of measuring (E3), during a test period (P2) after the reference period (P1), a second chemical signal (S1 _{P2}) by means of the sensor to be calibrated (1) and a second reference signal (S2_{P2}) by means of the reference sensor (2, 2'),
- at least one step of determining (E4) a second regression function (g_{P2}) defined as follows: S2_{P2} = g_{P2}(S1_{P2}),
- at least one step of calculating (E5) a difference (ε) between the first regression function (g_{P1}) and the second regression function (g_{P2}) and
- when the difference (ε) is greater than a reference difference (ε_{ref}), at least one step of determining (E6) an optimised conversion function (f*) so that the second regression function (g_{P2}) is substantially equal to the first regression function (g_{P1}), so as to calibrate the sensor to be calibrated (1).
